(19) Europäisches Patentamt / European Patent Office / Office européen des brevets

(11) **EP 3 045 574 B1**

(12) **EUROPEAN PATENT SPECIFICATION**

(45) Date of publication and mention
of the grant of the patent:
**14.02.2018 Bulletin 2018/07**

(51) Int Cl.:
*D01F 6/62* (2006.01)          *A61L 31/06* (2006.01)

(21) Application number: **14843446.7**

(22) Date of filing: **11.09.2014**

(86) International application number:
**PCT/JP2014/074106**

(87) International publication number:
**WO 2015/037671 (19.03.2015 Gazette 2015/11)**

(54) **ULTRAFINE POLYESTER FIBER**

ULTRAFEINE POLYESTERFASER

FIBRE POLYESTER ULTRAFINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2013 JP 2013189574**

(43) Date of publication of application:
**20.07.2016 Bulletin 2016/29**

(73) Proprietor: **ASAHI KASEI KABUSHIKI KAISHA Tokyo 101-8101 (JP)**

(72) Inventors:
 • **TOYODA, Keiichi**
  **Tokyo 101-8101 (JP)**
 • **TAKAHASHI, Tetsuko**
  **Tokyo 101-8101 (JP)**
 • **KOJIMA, Junichi**
  **Tokyo 101-8101 (JP)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
**EP-A1- 1 498 520          EP-A1- 1 584 711
EP-A1- 2 826 894          WO-A1-2012/102311
WO-A1-2013/137263          JP-A- H09 316 726
JP-A- H11 501 243          JP-A- 2003 041 432
JP-A- 2004 113 441          JP-A- 2010 196 227**

Remarks:
The file contains technical information submitted after the application was filed and not included in this specification

**Description**

Technical Field

The invention relates to an ultrafine polyester fiber that is suitable as a material for implantation into the human body.

Background Art

Polyester fibers composed mainly of polyethylene terephthalate (hereunder abbreviated as "PET") are widely used as constituent materials of stent graft fabrics and bioimplantable medical equipment such as artificial blood vessels.

A stent graft is an artificial blood vessel-like device equipped with a spring-like metal tubular fabric known as a stent (hereunder also referred to as "stent graft fabric" or "graft"), and such devices are used for treatment of aortic aneurysms. Transcatheter intravascular treatment using stent grafts (a method of treatment in which a narrow catheter having a stent graft compressively inserted therein is introduced through the artery at the base of the foot, and the stent graft is opened and fixed at the site of aneurysm, whereby blood flow into the aneurysm is blocked and rupture of the aneurysm is prevented), does not involve thoracotomy or laparotomy as with artificial blood vessel replacement, and therefore in recent years its application has been rapidly increasing, as it helps to reduce physical and economical burden.

Recently, there has been a rapid increase in the demand for smaller-diameter stent grafts in order to reduce the physical burden on patients with stent grafts or to widen the scope of patients that can be treated, and therefore the development of stent graft fabrics with narrower wall thicknesses (thin-walled fabrics) are being anticipated for use as stent graft members.

Polyester fibers used in conventional stent graft fabrics have single fiber finenesses exceeding 10 $\mu$m fiber sizes, the fibers employed having a large total fineness (product of the single fiber fineness and number of filaments), and therefore the use of polyester fibers with smaller total fineness and single fiber fineness, i.e. the use of ultrafine polyester fibers, is desired with the expectation of allowing stent graft fabrics with narrower wall thicknesses to be obtained.

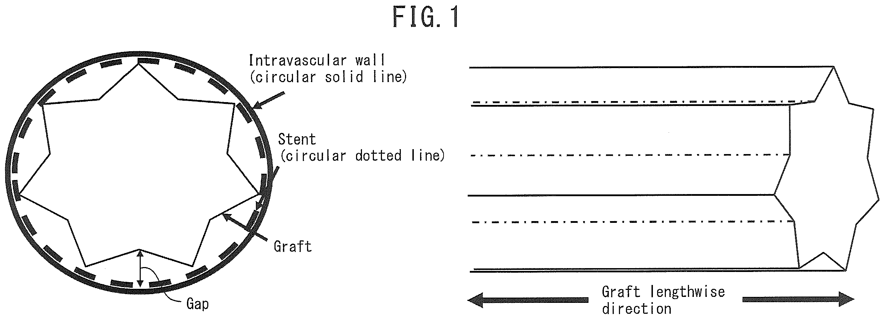

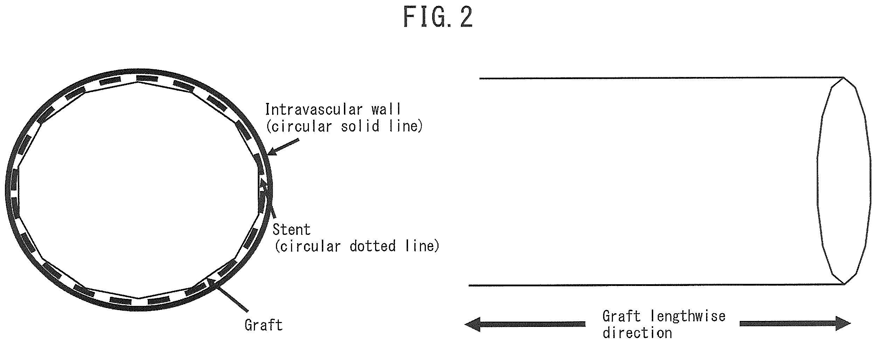

However, narrowing the wall thickness of a stent graft fabric raises a major issue. When a stent graft fabric (graft) is sutured with a metal stent and suture thread to finish the stent graft as a final product, a thin wall thickness of the stent graft fabric notably increases the flexibility of the fabric, resulting in difficult handleability during suturing. Consequently, integration between the stent and graft is poor and can create gaps between the intravascular walls and the graft, leading to concerns of blood leakage (endoleakage) (see Figs. 1 and 2). Blood leakage is a major problem encountered in stent graft interpolation surgery, and when blood leakage occurs it may become impossible to halt blood flow to the aneurysm, depending on the location. Furthermore, poor integration between the stent and graft is associated with serious problems such as tearing of sutured sections with the stent during actual use (in an intravascular pulsating environment).

Therefore, in order to meet the needs of medical locales for smaller stent graft diameters, it is necessary to improve integration between the stent and graft.

Ultrafine polyester fibers include composite spun ultrafine polyester fibers obtained using a polymer other than a PET component and a solvent, and direct spun ultrafine polyester fibers obtained using only PET polymers, but from the viewpoint of biological safety as a material for implantation into the human body, it is preferred to use direct spun polyester fibers that do not introduce concerns regarding residue (of polymers other than PET, of hydrolyzable monomers of the polymer, and of the solvent, etc.).

Patent Documents 1 to 3 listed below disclose direct spun ultrafine polyester fibers. The present inventors have used such conventional direct spun ultrafine polyester fibers to produce tubular woven fabrics, scouring and heat setting them, and then subsequently combining them with a Z-shaped stent and conducting sterilization by a known method, to produce a model stent graft final product. However, as feared, the problems of loosening between the stent and graft during suturing, and loosening even after the final sterilization, still remained.

Thus, it is the current state of affairs that ultrafine polyester fibers capable of meeting needs (reduced diameters) in medical environments and solving the associated problems have not yet been obtained for use as constituent materials of bioimplantable medical equipment such as stent graft fabrics.

Prior Art Documents

Patent Documents

Patent Document 1: Japanese Unexamined Patent Publication (Kokai) SHO No. 55-1338

Patent Document 2: Japanese Unexamined Patent Publication (Kokai) SHO No. 55-132708
Patent Document 3: Japanese Unexamined Patent Publication (Kokai) No. 2006-132027

SUMMARY OF THE INVENTION

Problems to be Solved by the Invention

The problem to be solved by the invention is to provide ultrafine polyester fibers capable of both meeting needs (reduced diameters) in medical environments and solving the associated problems (stent and graft integration) for use as constituent materials of bioimplantable medical equipment such as stent graft fabrics.

Means for Solving the Problems

As a result of much research and experimentation, the present inventors have found that the thermal shrinkage stress of ultrafine polyester fibers composing a stent graft fabric is strongly correlated with integration with the stent, and the invention has been completed upon this finding.
Specifically, the present invention is as follows.

[1] An ultrafine polyester fiber having a polyethylene terephthalate component content of 98 wt% or greater, and satisfying the following features:

(1) a reduced viscosity ($\eta$sp/c) of 0.80 dl/g or greater,
(2) a total fineness of 7 dtex or greater and 120 dtex or less, and a single fiber fineness of 0.5 dtex or less,
(3) a maximum thermal shrinkage stress of 0.05 cN/dtex or greater in a temperature range of between 80°C and 200°C, and
(5) a birefringence of 0.20 or greater in the region spreading from the surface of the fiber to a depth of 0.1 $\mu$m.

[2] The ultrafine polyester fiber according to [1], further satisfying the following condition:

(4) a degree of crystallinity of 35% or greater in the region spreading from the surface of the fiber to a depth of 0.1 $\mu$m.

[3] A fabric comprising at least 20 wt% of the ultrafine polyester fiber according to [1] or [2].

[4] A stent graft fabric comprising at least 20 wt% of the ultrafine polyester fiber according to [1] or [2].

[5] A stent graft comprising the stent graft fabric according to [4].

6. An artificial fiber fabric comprising at least 20 wt% of the ultrafine polyester fiber according to [1] or [2].

Effects of the Invention

The ultrafine polyester fiber of the invention does not invite concerns arising with residue from components other than PET as with composite spun ultrafine polyester fibers, and it can therefore ensure the necessary biological safety as a material for implantation into the human body. In addition, since the ultrafine polyester fiber of the invention is very fine (both total fineness and single fiber fineness) and has a high thermal shrinkage stress, it can both satisfy the needs in medical environments for small stent graft diameters, and solve the problem of improved integration with the stent. Furthermore, since the ultrafine polyester fiber of the invention has a surface layer section with a high degree of crystallinity and high orientation, it is possible to ensure long-term stability in the body, which is an issue when using ultrafine polyester fibers.

Brief Description of the Drawings

Fig. 1 is a schematic diagram showing a gap produced between an intravascular wall and a graft, where integration between the stent and graft has become impaired.
Fig. 2 is a schematic diagram showing a gap produced between an intravascular wall and a graft, where integration between the stent and graft is satisfactory.

Fig. 3 is a temperature-thermal shrinkage stress curve for an ultrafine polyester fiber ((Reference) Example 1).

Fig. 4 is a temperature-thermal shrinkage stress curve for an ultrafine polyester fiber ((Reference) Example 2).

Fig. 5 is a temperature-thermal shrinkage stress curve for an ultrafine polyester fiber ((Reference) Comparative Example 1).

Fig. 6 is a temperature-thermal shrinkage stress curve for an ultrafine polyester fiber ((Reference) Comparative Example 2).

Embodiments for Carrying Out the Invention

A preferred embodiment of the invention (hereunder referred to as "the present embodiment") will now be explained in detail.

The ultrafine polyester fiber of the present embodiment must have a PET component content of 98 wt% or greater, or in other words, a content of less than 2 wt% of components other than PET. Here, "components other than PET" refers to components incorporated into the molecular chain by copolymerization or the like, or copolymerized PET, polyamide, polystyrene or copolymers of the same, adhered onto the surfaces of polyester fibers, polymers other than PET components used for production of composite spun ultrafine polyester fibers, such as polyethylene and polyvinyl alcohol, and decomposition products of these polymers. According to the invention, components other than PET do not include PET-derived monomers and oligomers such as ethylene glycol, terephthalic acid (TPA), monohydroxyethylene terephthalate (MHET) and bis-2-hydroxyethyl terephthalate (BHET). In addition, they do not include coating agents such as collagen or gelatin that are coated or impregnated into grafts for the purpose of increasing biocompatibility. If the content of components other than PET is 2 wt% or greater, the components will elute out into the body when embedded, potentially causing heat release or heterogenization reactions. The content of components other than PET in the ultrafine polyester fiber is preferably less than 1 wt%, more preferably less than 0.5 wt% and most preferably zero.

The reduced viscosity of the ultrafine polyester fiber of the present embodiment must be 0.80 dl/g or greater. There is a correlation between the reduced viscosity of an ultrafine polyester fiber and the thermal shrinkage stress, described hereunder, such that when the reduced viscosity of the ultrafine polyester fiber is less than 0.80 dl/g the thermal shrinkage stress of the ultrafine polyester fiber falls below 0.05 cN/dtex, making it impossible to solve the problem of integration with the stent. Furthermore, from the viewpoint of the rupture strength of the stent graft fabric, the tensile strength of the ultrafine polyester fiber, as the constituent fiber, is preferably 3.5 cN/dtex or greater, and for this purpose the reduced viscosity of the ultrafine polyester fiber is preferably as high as possible. Therefore, from the viewpoint of achieving the target thermal shrinkage stress value and tensile strength, the reduced viscosity of the ultrafine polyester fiber is preferably 0.82 dl/g or greater and more preferably 0.85 dl/g or greater. There is no particular restriction for the upper limit of the reduced viscosity of the ultrafine polyester fiber of the invention, on the other hand, but the reduced viscosity of the polyester fiber obtained by melt extrusion has a practical limit of 1.50 dl/g, and from the viewpoint of minimizing variation in size between monofilaments, it is preferably no greater than 1.30 dl/g and more preferably no greater than 1.20 dl/g.

The total fineness of the ultrafine polyester fiber of the present embodiment must be between 7 dtex and 120 dtex, inclusive, from the viewpoint of obtaining a narrow wall thickness for the stent graft fabric. The total fineness is the product of the size of a single monofilament (single fiber fineness) and the total number of filaments. For example, the thickest blood vessel in which a stent graft can be used is the thoracic aorta, with usually about 40 to 50 mm as the inner diameter.

For reduced physical burden for patients and a wider scope of applicable patients, in the thoracic aorta it is desirable for a stent graft with a maximum inner diameter of 50 mm to be insertable in a catheter of up to 18 French (6 mm inner diameter), but study by the present inventors to date has shown that the maximum thickness for a tubular fabric with an inner diameter of 50 mm that can pass through a 6 mm diameter hole is 90 $\mu$m, and since this thickness does not significantly change even when the inner diameter of the tubular fabric is varied, the standard for the thickness of the fabric is no greater than 90 $\mu$m for specifying the single fiber fineness and total fineness of the ultrafine polyester fiber to be used in a stent graft fabric.

If the total fineness of the ultrafine polyester fiber is less than 7 dtex, the thickness of the fabric becomes reduced allowing the requirement for a small stent graft diameter to be satisfied, but it cannot withstand actual fabric use due to blood leakage from the wall face or insufficient long-term durability. In addition, if the total fineness of the ultrafine polyester fiber exceeds 120 dtex the thickness of the fabric will exceed 90 $\mu$m even if the single fiber fineness is 0.5 dtex or smaller, for example, and it will not be able to pass through a 6 mm diameter hole (assuming a 6 mm inner diameter catheter), when formed into a tubular fabric with an inner diameter of 50 mm, for example. From the viewpoint of achieving both narrow wall thickness and practical performance of the fabric, therefore, the total fineness of the ultrafine polyester fiber is preferably between 10 dtex and 110 dtex, inclusive, and more preferably between 15 dtex and 100 dtex, inclusive.

On the other hand, the single fiber fineness of the ultrafine polyester fiber of the present embodiment must be no greater than 0.5 dtex from the viewpoint of achieving an extremely thin thickness for a stent graft fabric. The single fiber fineness is the size per monofilament. If the single fiber fineness exceeds 0.5 dtex, it will be difficult to achieve a narrow wall thickness with a fabric thickness of 90 μm or less even if the total fineness is 120 dtex or smaller. Also, if the single fiber fineness is 0.5 dtex or smaller, the increased affinity with vascular endothelial cells will promote integration between the vascular wall tissue and the fabric, thus helping to prevent movement and separation of the stent graft inside the vessel, and inhibiting production of thrombi. From the viewpoint of the fabric narrow wall thickness and cellular affinity, the single fiber fineness of the ultrafine polyester fiber is preferably no greater than 0.4 dtex and more preferably no greater than 0.3 dtex. There is no particular restriction on the lower limit for the single fiber fineness, but from the viewpoint of suitability for post-treatment steps such as textile processing and the rupture strength of the fabric, it is preferably 0.01 dtex or greater and more preferably 0.03 dtex or greater.

The ultrafine polyester fiber according to one embodiment must have a maximum thermal shrinkage stress value of 0.05 cN/dtex or greater in a temperature range of between 80°C and 200°C, from the viewpoint of improving integration between the stent and graft. The fibers composing the stent graft fabric are subjected to a heat setting step in a temperature range of 160°C to 190°C during the molding process for the stent graft fabric (tubular woven fabric). A stent graft is produced via a sterilization step such as autoclave sterilization (110 to 120°C), dry air sterilization (180 to 190°C) or the like, and the fibers immediately after spinning have a reduced thermal shrinkage stress value due to their thermal history. According to the invention, integration between the stent and graft is achieved because the ultrafine polyester fibers composing the final product after passing through these steps retain a maximum thermal shrinkage stress of 0.05 cN/dtex or greater in a temperature range of between 80°C and 200°C.

Fibers retaining high residual stress at a temperature of below 80°C, as the glass transition point of PET, undergo structural changes with time due to the product storage environment, which can lead to problems such as deformation of the stent graft. In a temperature range exceeding 200°C, on the other hand, there is no correlation between thermal shrinkage stress and integration between the stent and graft. Furthermore, when the thermal shrinkage stress is less than 0.05 cN/dtex, i.e. when the residual stress is low, there is no integration with the stent but rather fold-like gaps are generated in the lengthwise direction, as shown in Fig. 1, leading to the serious problem of blood leakage after implantation. The thermal shrinkage stress in the temperature range of between 80°C and 200°C is preferably 0.08 cN/dtex or greater and more preferably 0.1 cN/dtex or greater. There is no particular restriction on the upper limit for the thermal shrinkage stress in the temperature range of between 80°C and 200°C, but from the viewpoint of maintaining uniformity of the woven density, it is preferably less than 1 cN/dtex.

The ultrafine polyester fiber according to another embodiment has a degree of crystallinity of 35% or greater in the region from the fiber surface to a depth of 0.1 μm, from the viewpoint of long-term durability when embedded in the body. One aspect of long-term durability when embedded in the body is resistance to hydrolysis, and there is a correlation with the degree of crystallinity on the surface layer section of the fibers in contact with blood or body fluids, the hydrolysis being inhibited if the degree of crystallinity is 35% or greater in the region from the fiber surface to a depth of 0.1 μm, thus allowing the physical properties to be maintained in the body over extended periods. The degree of crystallinity of the ultrafine polyester fiber in the region from the fiber surface to a depth of 0.1 μm is preferably 38% or greater and more preferably 40% or greater, from the viewpoint of long-term durability.

Likewise, from the viewpoint of long-term durability (hydrolysis resistance) when embedded in the body, the ultrafine polyester fiber of the present embodiment has a birefringence $\Delta n_s$, in the region from the fiber surface to a depth of 0.1 μm, of 0.200 or greater, more preferably 0.220 or greater and most preferably 0.240 or greater.

The ultrafine polyester fiber of the present embodiment preferably has a tensile strength of 2.5 cN/dtex or greater and a tensile elongation of 12% or greater. If the tensile strength of the ultrafine polyester fiber is 2.5 cN/dtex or greater, it will be possible to exhibit excellent mechanical/physical properties as a stent graft fabric. On the other hand, increasing the draw ratio for a polyester fiber can increase the tensile strength, but even if the tensile strength is increased to 2.5 cN/dtex or greater by stretching, for example, a tensile elongation of less than 12% will lead to inferior toughness, and tearing or breakage in response to impacts or prolonged pulsation. From the viewpoint of long-term durability of the graft, therefore, the tensile strength of the ultrafine polyester fiber of the invention is more preferably 3.0 cN/dtex or greater and even more preferably 3.5 cN/dtex or greater. From the same viewpoint, the tensile elongation of the ultrafine polyester fiber of the present embodiment is more preferably 15% or greater and even more preferably 20% or greater.

The ultrafine polyester fiber of the present embodiment also effectively functions as constituent fiber for materials for implantation into the human body other than stent graft fabrics, such as artificial blood vessels, artificial fiber fabrics, antiadhesive agents, artificial valves and the like. In addition, the ultrafine polyester fiber of the invention effectively functions as constituent fiber for materials for medical use other than materials for implantation into the human body, such as external hemofiltration materials, cell separating membranes, cell adsorption materials and cell culturing substrates. Naturally, the ultrafine polyester fiber of the present embodiment also effectively functions as constituent fiber for non-medical materials, including clothing materials, filters, wiping materials and the like.

The ultrafine polyester fiber of the present embodiment effectively functions as constituent fiber for a stent graft fabric. Fabrics for use in a stent graft according to the invention are preferably woven fabrics, from the viewpoint of exhibiting strength and preventing blood leakage. Moreover, from the viewpoint of a more narrow wall thickness of the fabric, the woven fabric of the invention must be composed of at least 20 wt% of the ultrafine polyester fiber of the present embodiment. If the component proportion ratio of ultrafine polyester fiber of the present embodiment in the woven fabric is less than 20 wt%, the thickness of the fabric will exceed 90 $\mu$m, and it will be difficult to achieve a small diameter for the stent graft as the final product. Furthermore, a component proportion ratio of less than 20 wt% for the ultrafine polyester fiber will result in inferior integration with the stent. In a woven fabric for the present embodiment, the component proportion ratio of ultrafine polyester fiber of the present embodiment is preferably 25 wt% or greater, more preferably 30 wt% or greater and most preferably 35% or greater. The superfine fiber of the present embodiment may be used for either the warp yarn or weft yarn of the woven fabric, or for both, but it is most preferably used for the weft yarn from the viewpoint of improved integration between the stent and graft.

The materials other than the ultrafine polyester fiber composing the woven fabric of the present embodiment may be polyester fiber, polyamide fiber, polyethylene fiber, polypropylene fiber or the like, that are not within the scope of the invention. These may be monofilaments or multifilaments, and one type or a combination of two or more types of fiber material may be used according to the purpose, where combinations may be composite fibers comprising polyester fiber of the present embodiment twisted with other fibers, or using other fibers as the warp yarn or weft yarn of a woven fabric, or using them partially in certain sections.

A stent graft fabric may be a sheet-like fabric attached together into a tubular form, but the thickness will increase at the attachment sections and it will not be possible to fold the fabric in a narrow manner, and therefore it is preferably a tubular seamless woven fabric. A tubular seamless woven fabric is also preferred because having the weft yarn composed of continuous ultrafine polyester fibers will improve integration between the stent and graft. The fabric structure may be a plain weave, twill weave, satin weave or the like without any particular restrictions, but from the viewpoint of obtaining a narrow wall thickness for the fabric and preventing blood leakage, it preferably has a plain weave structure or twill weave structure. The warp density and weft density of the tubular seamless woven fabric of the invention is preferably 39.4/cm (100/inch) or greater and more preferably 47.2/cm (120/inch) or greater from the viewpoint of preventing blood leakage. The upper limit is not particularly restricted but is essentially no greater than 138/cm (350/inch).

The thickness of the woven fabric of the present embodiment is between 10 $\mu$m and 90 $\mu$m, inclusive, preferably between 15 $\mu$m and 80 $\mu$m, inclusive, and more preferably between 20 $\mu$m and 70 $\mu$m, inclusive, from the viewpoint of obtaining a smaller diameter. The thickness of the woven fabric is defined as the average of the measured values for the thickness of the fabric at 10 locations arbitrarily selected within a range in the circumferential direction of the tubular woven fabric (arbitrarily depending on the diameter) and the lengthwise direction (10 cm to 30 cm), using a thickness gauge. If the thickness of the fabric exceeds 90 $\mu$m, it will not be possible for a tubular woven fabric with an inner diameter of 50 mm, for example, to pass through a hole with a diameter of 6 mm. On the other hand, if the fabric thickness is smaller than 10 $\mu$m it will not be possible to maintain sufficient rupture strength. For measurement of the thickness of the woven fabric, the values for the thickness variation Z at the measurement points, represented by the following formula (1):

$$Z\ (\%)\ =\ (Z_{av}\ -\ Z_i)/Z_{av}\ \times\ 100 \qquad formula\ (1)$$

wherein $Z_{av}$ is the average for 10 measured values, $Z_i$ is the measured value at each point and i is an integer of 1 to 10. are all preferably within ±15%.

If the thickness variation is greater than -15%, passage through a 6 mm-diameter hole may not be possible even if the average value for the fabric thickness is 90 $\mu$m or smaller. Also, sections with thickness variation exceeding 15% may have low thickness and impaired rupture strength and water permeation prevention. The thickness variation Z is preferably within ±12%, and most preferably within ±10%.

The outer diameter of the woven fabric of the invention will depend on the inner diameter of the blood vessel in which the stent graft is to be used, and may be between 6 mm and 50 mm, inclusive.

The woven fabric of the present embodiment has a water permeability of no greater than 300 cc/cm$^2$/min before and after needle penetration. The water permeability of the fabric is an index of blood leakage prevention, and with a water permeability of no greater than 300 cc/cm$^2$/min, blood leakage from the fabric wall face will be minimized. On the other hand, the stent graft fabric may be prepared as a final stent graft product sewn together with a metal stent using suture thread, but if large needle holes are opened in the fabric during such a procedure, blood leakage may occur at those locations. In other words, the water permeability after penetration of a needle must be no greater than 300 cc/cm$^2$/min, for practical performance as a stent graft fabric. The water permeability after needle penetration is the value measured after passing a tapered 3/8 needle 10 times through the fabric, in an arbitrary 1 cm$^2$ area. Since ultrafine polyester fiber is used in the tubular seamless woven fabric of the present embodiment, the monofilaments are pressed flat in the woven texture to fill the gaps at the crossing points of the warp yarn and weft yarn, and the water permeability before needle penetration is kept to a minimum. Also, as regards the water permeability after needle penetration, in a fabric having polyester fiber of normal thickness, having monofilament diameters of several $\mu$m or greater, woven to high density or a strongly calender pressed fabric, designed to minimize water permeability, the fibers composing the fabric are firmly constrained (mobility of the individual fibers is inhibited), and therefore the fibers that have moved when the needle passes through are inhibited from returning to their original positions, and open needle holes remain after needle penetration. However, since the woven fabric of the present embodiment employs ultrafine polyester fiber composed of numerous superfine filaments, it is resistant to formation of needle holes and the water permeability after needle penetration can be limited to no greater than 300 cc/cm$^2$/min. From the viewpoint of practical performance, the water permeability of the tubular seamless woven fabric of the present embodiment before and after needle penetration is no greater than preferably 250 cc/cm$^2$/min and more preferably 200 cc/cm$^2$/min.

The woven fabric of the present embodiment must have a rupture strength of 100N or greater as measured by a rupture strength test according to ANSI/AAMI/IS07198: 1998/2001. If the rupture strength of the fabric is less than 100N, this may constitute a problem in terms of safety when used as a stent graft fabric, considering rupture by expanding force of the stent, for example, and it is preferably 120N or greater and more preferably 140N or greater. There is no particular restriction on the upper limit for the rupture strength of the fabric, but from the viewpoint of balance with narrow wall thickness of the fabric, it is essentially no greater than 500N.

The tubular seamless woven fabric of the present embodiment may be coated with collagen, gelatin or the like in a range that is within the conditions of thickness and outer diameter specified by the invention.

The woven fabric of the present embodiment is used as a stent graft, in combination with a stent (spring-like metal) that is to serve as an inflatable member.
The type of stent graft may be a tubular simple straight type, or a branched type or fenestrated type suitable for branched blood vessels, for use mainly in the abdominal region. An inflatable member may employ a self-inflating material using a shape memory alloy, superelastic metal or synthetic polymer material. An inflatable member may have any design of the prior art. An inflatable member can also be applied as a type that expands with a balloon, instead of a self-inflating type. A stent graft according to a preferred mode of the invention has a gap size of preferably no greater than 2 mm between the stent and graft. More specifically, for example, when the final stent graft product is opened into a transparent glass tube (or acrylic tube) with the same diameter as the inflated diameter (outer diameter) of the stent, as shown in Fig. 1 or Fig. 2, preferably there are no sections exceeding a maximum length of 2 mm in the gaps produced between the inner diameter of the stent and the graft.

A stent graft according to a preferred embodiment of the invention is inserted into a catheter and delivered into a blood vessel. The stent graft of the present embodiment is thin, with a fabric thickness of 90 $\mu$m or smaller, with high flexibility, and it can therefore be inserted into a narrow-diameter catheter, and consequently can be easily delivered into blood vessels, with low risk of damage to vascular walls. The catheter used is preferably one of the prior art, such as a tube type or balloon type. Also, a stent graft inserted into a narrow-diameter catheter for the invention can be delivered into and be indwelling in a blood vessel, using a conventional delivery system. When the tubular seamless woven fabric of the present embodiment is to be used as a stent graft fabric, the stent graft may have a narrow diameter, and it can therefore reduce the physical and economical burden on patients, such as shortening inpatient periods, and can reduce risks such as vascular wall damage. In addition, it is possible to widen the range of applications to cases that have hitherto been excluded as targets of transcatheter intravascular treatment, such as females and Asians that have narrower arteries.

The method for producing ultrafine polyester fiber according to the present embodiment will now be explained in greater detail, with the understanding that the invention is not limited to the methods described.

According to the invention, it is preferred to employ a direct melt spinning method in which a polymer composed essentially of polyethylene terephthalate (PET) is melt spun and then stretched to produce an ultrafine polyester fiber. The melt spinning machine used may be a known spinning machine equipped with a dryer, extruder and spinning head. The molten PET is discharged from a plurality of discharge nozzles mounted on the spinning head, and immediately after spinning it is blasted with cooling air from a cooling device provided under the spinneret surface for cooling to solidification, and spun into a multifilament.

For production of the ultrafine polyester fiber of the present embodiment, it is preferred to use a PET polymer with a reduced viscosity of 0.85 dl/g or greater from the viewpoint of exhibiting fiber strength and high toughness, but from the viewpoint of spinning stability the upper limit for the reduced viscosity of the starting PET polymer is 1.60 dl/g. From the viewpoint of physical properties and spinning stability of the ultrafine polyester fibers, the reduced viscosity of the starting PET polymer is more preferably between 0.87 dl/g and 1.50 dl/g inclusive, and more preferably between 0.90 dl/g and 1.40, inclusive. The starting PET polymer to be used for the invention is preferably produced using a polymerization catalyst other than the heavy metal antimony, from the viewpoint of biological safety. Preferred polymerization catalysts include compounds composed mainly of titanium, such as amorphous titanium oxide and organic titanium, or germanium which is used for polymerization of PET for food packaging films such as PET bottles. The starting PET polymer to be used for the present embodiment preferably has a lower content of crystalline titanium oxide used as a delustering agent, from the viewpoint of preventing elution in the body. Specifically, the amount of titanium element is preferably no greater than 3000 ppm, more preferably no greater than 2000 ppm and even more preferably no greater than 1000 ppm with respect to the polymer weight.

In the method for producing an ultrafine polyester fiber according to the present embodiment, preferably the spinneret surface temperature during spinning is controlled to a range of between 290°C and 320°C, and when the discharge nozzle is a multiple array, the spinneret surface temperature distribution (the temperature distribution from the outermost array to the innermost array) is preferably within 10°C. By controlling the spinneret surface temperature to a range between 290°C and 320°C, it is possible to minimize reduction in molecular weight by thermal decomposition of PET polymers with a relatively high polymerization degree, while simultaneously accomplishing spinning without size unevenness in the fiber axis direction. If the spinneret surface temperature is below 290°C the pressure of the spinpack will increase, producing melt fracture in the discharged yarn and increasing variation between monofilaments, and making it impossible to exhibit the desired strength. If the spinneret surface temperature exceeds 320°C, it may not be possible to exhibit the desired strength due to lower molecular weight induced by thermal decomposition in the spinpack, and spinneret contamination may render spinning impossible. By controlling the spinneret surface temperature distribution to within 10°C, on the other hand, it is possible to minimize variation in the melt viscosity of the discharge polymer and reduce monofilament diameter unevenness between monofilaments (interfilament variation). From the viewpoint of limiting variation in fiber size between monofilaments and size unevenness in the fiber axis direction, and also exhibiting strength, more preferably the spinneret surface temperature is between 295°C and 310°C, and the spinneret surface temperature distribution is controlled to within 5°C.

There are no particular restrictions on the means for controlling the spinneret surface temperature and the temperature distribution between nozzles to the ranges specified above, but a method of temperature adjustment by surrounding the lower spinneret portion with a heater, or a method of heating adjustment with a heater around the protruding spinneret, may be employed. In either of these methods, it is important to avoid heat from being transferred from the heater to the spinning head, from the viewpoint of inhibiting reduction in polymerization degree by thermal decomposition of the polymer in the spinning head, and from the viewpoint of high strength, high toughness and spinning stability of the ultrafine polyester fiber. Heat transfer from the heater can be blocked, for example, by not directly mounting the heater on the spinning head and inserting a heat-shielding plate between them, and this method is effective both when temperature adjustment is made by heating the lower part of the spinneret with a surrounding heater, and when heating is carried out around the protruding spinneret. Also, for heating of the protruding spinneret, heating only the protruding spinneret portion with an induction heating system is also effective for preventing heat transfer to the spinning head.

According to the present embodiment, the number of discharge nozzles per spinneret is preferably 20 to 1500 bored holes. The arrangement of the discharge nozzles is not particularly restricted and may be a circumferential arrangement, crossing arrangement or the like, but for a circumferential arrangement they are preferably in multiple circumferential rows in order to increase the number of nozzles. As mentioned above, according to the present embodiment the discharged yarn is cooled to solidification by blasting cooling air from a cooling device provided below the spinneret surface, but in the case of multiple circumferential rows, depending on the number of filaments and the number of rows, the blasted cooling air may not easily reach the innermost rows due to the influence of accompanying flow, and uneven cooling may occur in the discharged yarn between the outermost rows and the innermost rows, often resulting in high fiber size variation between the monofilaments (interfilament variation). In this case, a nozzle-free area is provided between the outermost rows and innermost rows of the spinneret, so that cooling air can more easily reach the innermost rows. In other words, it is preferred to provide a flow passage for the cooling air, so that cooling solidification of the discharged yarn is accomplished uniformly from the outermost rows to the innermost rows and interfilament variation is reduced. The number of rows in a multiple circumferential arrangement, the distance between rows, the distance between the discharge nozzles on circumferential rows, and the design of the cooling air flow passage may be determined as desired within ranges for the desired filament number and single fiber fineness and the allowable spinneret size, but the distance between circumferential rows is preferably between 1 mm and 12 mm, inclusive, from the viewpoint of preventing fusion between the monofilaments and avoiding an excessive spinneret size, and the distance between discharge nozzles on the circumference is preferably between 1.2 mm and 5 mm, inclusive, from the viewpoint of preventing uneven cooling, preventing fusion between the monofilaments, and achieving a suitable spinneret size design.

The hole diameter of the discharge nozzle is preferably between 0.05 mmφ and 0.15 mmφ, inclusive.

In the method for producing the ultrafine polyester fiber of the present embodiment, it is important to provide a hot zone in which the atmosphere temperature above and below the spinneret surface is controlled to 150°C or higher, and to pass the discharged yarn through it, from the viewpoint of high toughness and high crystallization and macromolecular orientation of the surface layer section, in which case the hot zone range is preferably located in a range of between 1 mm and 60 mm, inclusive, from the spinneret surface. The atmosphere temperature is the temperature at a point moved vertically downward at a spacing of 1 mm from the center section of the spinneret surface. Therefore, a hot zone of less than 1 mm cannot be measured. If the hot zone is greater than 60 mm, the yarn may slope and it will be difficult to wind up the filament. Even if the filament can be wound up, the interfilament variation and size unevenness (U%) in the fiber axis direction of the obtained ultrafine polyester fiber will be poor. Also, if the atmosphere temperature at the point 1 mm from the spinneret surface is not controlled to 150°C or higher, yarn bending will occur and spinning will not be possible, or even if it is possible, fibers with the desired strength will not be obtained. The hot zone conditions can be adjusted by the thickness and temperature of the heater mounted on the spinneret head, the elevation angle and temperature of the cooling air diffuser, and the thickness of the heat-shielding plate.

The hot zone is preferably within 50 mm and more preferably within 40 mm from the spinneret surface. If the hot zone environment is properly adjusted, it will be possible to use the heater described above for spinneret surface temperature control, and if blowing in of cooling air can be prevented, a heat-shielding plate with a thickness of 60 mm or smaller may be set in the spinning head.

In addition, from the viewpoint of spinning stability and controlling interfilament variation and size unevenness in the fiber axis direction, the discharged yarn is preferably quenched to solidification with a cooling system (described below) after passing through the hot zone, and the atmosphere temperature at the uppermost position of the cooling air blowing surface (a point 1 cm distant from the yarn discharged from the outermost row of the spinneret) is more preferably no higher than 120°C and even more preferably no higher than 100°C.

From the viewpoint of increasing spinning stability and minimizing interfilament variation between the ultrafine polyester fibers, it is important for the cooling air blowing device to be set surrounding the discharged yarn, and for variation Z in the cooling air speed from the cooling air blowing surface to be reduced. (The cooling air speed is measured on a 360° circumference from a specific location on the cooling air blowing surface with a 15° pitch, and the standard deviation of the cooling air speed for a total of 24 points is calculated as the variation Z for the cooling air speed.) The cooling air speed variation Z must be no greater than 0.15. If the cooling air speed variation Z exceeds 0.15, the yarn may slope and it may become difficult to wind up the filaments, and even if they can be wound up, the obtained ultrafine polyester fiber will have large yarn diameter variation between monofilaments. From the viewpoint of minimizing interfilament variation of the ultrafine polyester fiber, the cooling air speed variation Z is more preferably no greater than 0.13 and even more preferably no greater than 0.10. In addition, the cooling air speed is preferably between 0.6 m/s and 2.0 m/s from the viewpoint of uniformity of cooling from the outermost rows toward the innermost rows. Here, the cooling air speed is the average value of the cooling air speed measured at a total of 24 points for evaluation of the cooling air speed variation Z. If the cooling air speed is lower than 0.6 m/s it will be difficult for the blasted cooling air to reach the innermost rows, due to the influence of accompanying flow, and cooling unevenness will occur in the discharged yarn between the outermost rows and innermost rows, resulting in increased yarn diameter variation between monofilaments (interfilament variation). If the cooling air speed exceeds 2.0 m/s, on the other hand, the discharged yarn from the outermost rows may undergo swinging, resulting in yarn breakage, interfilament variation, and size unevenness in the fiber axis direction. The cooling air speed is more preferably between 0.7 m/s and 1.8 m/s, inclusive, and even more preferably between 0.8 m/s and 1.5 m/s, inclusive. The temperature of the cooling air is preferably controlled within a range of between -30°C and 18°C, from the viewpoint of quenching solidification and cooling uniformity of the discharged yarn, it being more preferably between -15°C and 16°C and even more preferably between -10°C and 15°C.

In the method for producing an ultrafine polyester fiber according to the present embodiment, preferably the discharged yarn is bundled at a location between 5 cm and 50 cm, inclusive, from the direct bottom of the spinneret, from the viewpoint of minimizing swinging of the yarn and increasing spinning stability, and it is more preferably between 10 cm and 40 cm, inclusive, and even more preferably between 15 cm and 30 cm, inclusive.

In the method for producing an ultrafine polyester fiber according to the present embodiment, spinning is preferably carried out at between 300 m/min and 3000 m/min, inclusive, supplying a finishing agent to the fiber bundle after bundling, from the viewpoint of spinning efficiency and high toughness, and this is more preferably between 700 m/min and 2800 m/min, inclusive, and even more preferably between 1000 m/min and 2500 m/min, inclusive. Also, from the viewpoint of bulk finishing and suitability for textile processing, the oil application rate of the finishing agent is preferably between 1 wt% and 3 wt%, inclusive, more preferably between 1.2 wt% and 2.8 wt%, inclusive, and even more preferably between 1.5 wt% and 2.5 wt%, inclusive.

In the method for producing an ultrafine polyester fiber according to the present embodiment, the unstretched yarn obtained by spinning at the speed mentioned above may be continuously stretched and taken up as a drawn yarn without first being wound up, or it may be first wound up as an unstretched yarn and then stretched on a separate line with a stretching/twisting machine, horizontal stretching machine or the like, and wound up as drawn yarn. In either case, preferably stretching is at a stretching temperature of 50°C to 120°C followed by heat treatment at 80°C to 180°C and wind-up, for a tensile elongation of 12% or greater.

In the method for producing an ultrafine polyester fiber according to the present embodiment, tangling treatment at the unstretched yarn stage or stretched yarn stage is preferred from the viewpoint of reducing fluff and yarn breakage during bulking treatment and textile processing, and the tangling treatment preferably employs a known tangling nozzle, with the number of tangles being in the range of 1 to 80/m and more preferably in the range of 5 to 50/m.

A woven fabric is produced using ultrafine polyester fiber obtained by the method described above, and from the viewpoint of ensuring a thermal shrinkage stress of 0.05 cN/dtex or greater for the ultrafine polyester fiber forming the fabric of the final stent graft product (following sterilization), the thermal shrinkage stress of the ultrafine polyester fiber used for weaving is preferably 0.2 cN/dtex or greater in a temperature range of between 80°C and 200°C.

An example of production of a tubular seamless woven fabric will now be described. The loom used to produce the tubular seamless woven fabric is not particularly restricted, and the use of a shuttle loom in which the weft yarn is passed through by reciprocal movement of a shuttle is preferred because it can minimize reduction in woven density at the tab sections of the woven fabric (the folded sections of the tubular woven fabric), and result in a uniform woven fabric thickness. When fibers with a relatively large single fiber fineness and total fineness are used to prepare a sack-like woven fabric with a large thickness and wide woven width, such as for an air bag, a shuttleless weaving machine such as an air jet loom, water jet room or rapier loom may be used, but when a low-thickness, high-density uniform woven fabric such as according to the invention is prepared with a shuttleless weaving machine, the woven density is notably decreased at the tab sections of the woven fabric causing partial increase in water permeability, and therefore blood leakage and the like become crucial defects when it is utilized as a stent graft fabric.

Also, for preparation of the tubular seamless woven fabric of the present embodiment, it is preferred to use a total surface temple for the purpose of stabilization before weaving, uniformity of the thickness and diameter of the woven fabric, and minimizing yarn breakage during processing. Since the tubular seamless woven fabric of the invention employs ultrafine polyester fiber and has a very thin thickness, when a total surface temple is used it preferably has a structure with minimal contact area between the woven fabric and total surface temple, or it is preferred to select a material with a low frictional coefficient for the total surface temple member at the section contacting with the woven fabric, for the purpose of minimizing abrasion of the woven fabric by the total surface temple. An appropriate design may be selected for the structure of the total surface temple and the frictional coefficient of the member, according to the single fiber fineness or total fineness of the ultrafine polyester fiber used and the woven density of the warp yarn or weft yarn.

When the tubular seamless woven fabric is prepared, it is necessary to control raising and lowering of the warp yarn, and for this purpose the apparatus used may be a Jacquard opening apparatus or dobby opening apparatus.

After weaving, the fabric is subjected to scouring treatment to remove oil solutions and the like and heat setting to stabilize the shape, there being no particular restrictions on the scouring temperature, treatment time, heat setting temperature or treatment time, or on the tension applied during these steps, which may be appropriately set so that the thermal shrinkage stress of the ultrafine fibers after sterilization is 0.05 cN/dtex or greater, in combination with the stent.

The treated woven fabric and stent are combined using suture thread. The conditions for joining the woven fabric and stent may be selected as appropriate for the shape of the stent. There are also no particular restrictions on the needle used for suturing, but preferably one is selected so that the water permeability after needle penetration is no greater than 300 cc/cm$^2$/min. The stent graft obtained by this method is subjected to sterilization treatment. The conditions for sterilization treatment are not particularly restricted, and are sufficient if selected for balance between the sterilization effect and the thermal shrinkage stress of the treated ultrafine polyester fiber.

Examples

The present invention will now be explained in more specific detail, with the understanding that the invention is in no way limited by the following examples. The major values for the physical properties were measured by the following methods.

(1) Reduced viscosity ($\eta$sp/c)

The reduced viscosity ($\eta$sp/c) is measured in the following manner.

- A dilute solution of 0.35 g of polyethylene terephthalate (PET) sample dissolved in 0.25 deciliter of 1,1,1,3,3,3-hexafluoro-2-propanol (HFIP) is prepared at room temperature.
- A Ubbellohde viscosity tube (tube diameter: 0.03) is used to measure the number of seconds of dropping of the dilute solution and HFIP solvent at 25°C, and the relative viscosity ($\eta$sp) is determined.
- The relative viscosity ($\eta$sp) is divided by the polymer concentration C (g/dl) and the reduced viscosity $\eta$sp/c is calculated.

(2) Component content P other than PET

(a) Content $P_1$ of residual components adhering on fiber surface

After cutting to a length of 1 cm for fiber or cutting to a 1 cm-square for a fabric, it is loosened into a fibrous form and scoured for 30 minutes with hot water at 95°C to remove the spinning oil solution and then dried at 105°C for 3 hours, and the weight ($W_0$) is measured. The fibrous substance is treated at 80°C x 45 minutes with a 3% sodium hydroxide aqueous solution with a liquor to goods ratio of 100, subjected to filtration and rinsing with purified water, repeated 3 times, and dried at 105°C $\times$ 3 hours, and the weight ($W_1$) is measured, and then the content of residual components adhering to the fiber surface is calculated by the following formula (2):

$$P_1 \ (wt\%) \ = \ (W_0 - W_1)/W_0 \ \times \ 100 \qquad Formula \ (2).$$

(b) Content $P_2$ of residual components adhering to surface even after treatment in (a), and/or components copolymerized with PET

The fibrous substance treated in (a) was dissolved in d-1,1,1,3,3,3-hexafluoro-2-propanol to 1-2 vol% (room temperature) and measured using $^1$H-NMR (AVANCEII AV400 M by Bruker BioSpin K.K.). The presence of signals other than for the PET component is confirmed from the NMR chart, and when a signal other than for the PET component is observed, the fiber surface-adhering component and/or copolymerizing component is identified and the content ($P_2$) is calculated from the NMR chart.

The value from (a) and (b) are summed to obtain the content P for components other than PET.

(3) Total fineness/single fiber fineness

The total fineness (dtex) is the value obtained by winding the fiber bundle 50 times around a skein with a 1 m circumference, measuring the weight of the yarn and multiplying the value by 200. The single fiber fineness (dtex) is the value of the total fineness determined by the method described above, divided by the filament number.

(4) Tensile strength and tensile elongation

The tensile strength and tensile elongation were measured according to JIS-L-1013.

(5) Thermal shrinkage stress

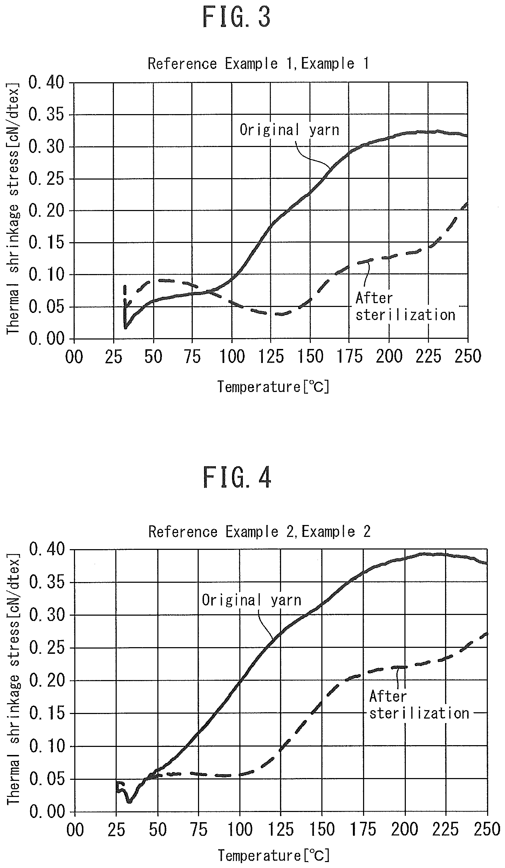

A thermal stress meter (KE-2S, by Kanebo Engineering Co.) was used for measurement of the thermal shrinkage stress. A fiber sample was connected into a ring with a circumference of 100 mm, and set on an upper hook and lower hook, leaving a spacing of 50 mm. The distance between the hooks was microadjusted to an initial load of 0.05 cN/dtex, and the temperature was increased from 30°C to 260°C at a temperature-elevating rate of 150°C/min, while keeping a constant length. The stress generated by the fiber sample was recorded during this time, and a temperature-thermal shrinkage stress curve was drawn by plotting temperature on the abscissa and stress on the ordinate, as shown in Figs. 3 to 6. The maximum thermal shrinkage stress between 80°C and 200°C was read off and used as the thermal shrinkage stress.

(6) Degree of crystallinity in region from the surface of the fiber to a depth of 0.1 $\mu$m

In order to determine the degree of crystallinity in the region spreading from the surface of the fiber to a depth of 0.1 $\mu$m, alkali etching treatment was carried out in the region spreading from the surface of the fiber to a depth of 0.1 $\mu$m by the method described below, and the degree of crystallinity of the region at a depth of 0.1 $\mu$m from the fiber surface was calculated from the degree of crystallinity before and after alkali etching treatment.

(Alkali etching method)

The weight was measured using ultrafine polyester fiber that had been moisture-controlled by air-drying for at least one day and one night in a steady temperature and humidity room controlled to a temperature of 23°C and a humidity of 50%. (Weight = $Y_0$.) It was immersed for a prescribed time period in a 1.9 mol/L potassium hydroxide aqueous solution containing 0.1 wt% cetyltrimethylammonium bromide as a hydrolysis accelerator, and alkali etching treatment was performed. The sample was then removed out and thoroughly rinsed with a 0.1 mol/L hydrochloric acid aqueous solution and purified water, and again moisture-controlled by air-drying for at least one day and one night in a steady temperature and humidity room, and the weight after alkali etching treatment was measured. (Weight = $Y_1$.) The weight retention before and after alkali etching treatment is expressed as $Y_1/Y_0$. Weight retention with alkali etching has a different weight reduction speed depending on the single fiber fineness of the fibers and the liquor to goods ratio, but it can be easily controlled by varying the immersion time in the alkali solution. Also, the weight retention in response to alkali treatment in the region spreading from the surface of the fiber to a depth of 0.1 $\mu$m can be calculated from the weight retention of the single fiber fineness of the fibers.

For example, in the case of ultrafine fibers with a single fiber fineness of 0.13 dtex, the weight retention before and after alkali etching treatment in the region to 0.1 $\mu$m from the fiber surface layer is 89%.

(Method of measuring degree of crystallinity)

The measurement was performed using a DSC (Pyris1, by Perkin Elmer). An approximately 5 mg portion of ultrafine polyester fiber sample was sealed in an aluminum sample container, and the DSC curve was measured under a nitrogen stream at a temperature-elevating rate of 20°C/min. Indium was used as the standard substance. The degree of crystallinity was calculated by the following formula (3):

$$\text{Degree of crystallinity (\%)} = (\text{Heat of fusion} - \text{cold crystallization heat})/(\text{equilibrium heat of fusion}) \times 100 \quad \text{formula (3)}.$$

The value used for equilibrium heat of fusion was 140 J/g.

(Degree of crystallinity in region to depth of 0.1 $\mu$m from surface layer)

From the degree of crystallinity before and after alkali etching treatment, with weight retention $Y_1/Y_0$, a degree of crystallinity $X_s$ of the region to a depth of 0.1 $\mu$m from the surface layer was calculated by the following formula (4):

$$X_s \ (\%) \ = \ (X_t - Y_1/Y_0 \times X_c) \div (1 - Y_1/Y_0) \quad \text{formula (4)}$$

whrein $X_s$ is the degree of crystallinity in the region to a depth of 0.1 $\mu$m from the surface layer, $X_t$ is the degree of crystallinity before alkaline etching, and $X_c$ is the degree of crystallinity after alkali etching, with weight retention $Y_1/Y_0$.

(7) Birefringence ($\Delta n_s$) in region up to a depth of 0.1 $\mu$m from the surface layer

Alkali etching treatment was performed in the region up to a depth of 0.1 $\mu$m from the surface layer by the same method as in (6) above, and the birefringence before and after alkali etching treatment was used to calculate the birefringence $\Delta n_s$ in the region up to a depth of 0.1 $\mu$m from the surface layer by the following formula (5):

$$\Delta n_s \ (\%) \ = \ (\Delta n_t - Y_1/Y_0 \times \Delta n_c) \div (1 - Y_1/Y_0) \quad \text{formula (5)}$$

wherein $\Delta n_s$ is the birefringence in the region to a depth of 0.1 $\mu$m from the surface layer, $\Delta n_t$ is the birefringence before alkaline etching, and $\Delta n_c$ is the birefringence after alkali etching, with weight retention $Y_1/Y_0$.

(Method of measuring birefringence $\Delta n$)

The birefringence $\Delta n$ was measured using a polarizing microscope (BX51 by Olympus Corp.) and a thick Berek compensator (U-CTB by Olympus Corp.), by a method according to "Fiber Handbook - Starting materials", p969 (5th printing, 1978, Maruzen Publishing), based on the retardation of polarized light on the fiber surface and the fiber size.

[Reference Examples 1 and 2]

Polyethylene terephthalate was used as the starting material, and melt spinning was performed to wind up 29 dtex/150F unstretched yarn.

The properties of the PET starting material were as follows.

Reduced viscosity ($\eta$sp/c = dl/g): Listed in Table 1 below.
Titanium content: 2 ppm
Diethylene glycol content: 0.8 wt%

Oligomer content: 1.2 wt%

The spinneret used was a spinneret having 3 rows with 50 discharge nozzles (hole diameter: 0.08 mmφ) bored in a circumferential manner per circle (each with 50 discharge nozzles) (number of nozzles: 150). Cooling of the yarn was accomplished basically using a cooling air blasting apparatus with an air diffuser at an elevation angle of 37°.

Spinning was otherwise carried out under the conditions described in Table 1, and 29 dtex unstretched yarn was taken up for 2 hours at 2000 m/min. Wind-up of the unstretched yarn was possible in a stable manner without any particular generation of yarn breakage or the like. The obtained unstretched yarn was subjected to hot-rolled stretching with a stretching machine having a publicly known heated roll, with a first roll temperature of 80°C and a second roll temperature of 130°C, to a draw ratio of 1.45, to obtain ultrafine polyester fiber. The content of components other than PET in the obtained ultrafine polyester fiber was less than 2 wt% in all cases. The reduced viscosity and physical properties of the obtained fiber are shown in Table 2 below. Also, the temperature-thermal shrinkage stress measurement curves are shown in Fig. 3 and Fig. 4.

[Reference Comparative Examples 1 and 2]

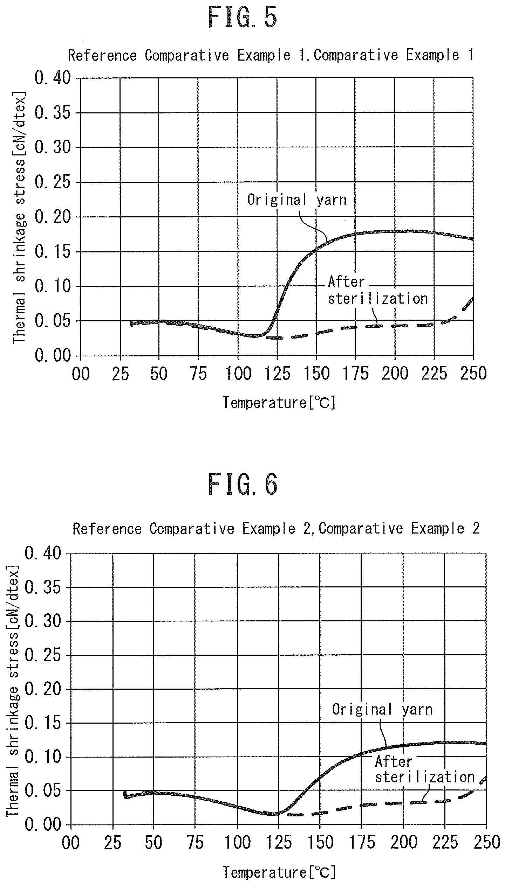

Spinning and stretching were carried out in the same manner as Examples 1 and 2 below to obtain ultrafine polyester fibers, except that the starting materials with reduced viscosity as listed in Table 1 were used and the spinneret surface temperature during spinning was controlled to the conditions listed in Table 1. The content of components other than PET in the obtained ultrafine polyester fiber was less than 2 wt% in all cases. The reduced viscosity and physical properties of the obtained fiber are shown in Table 2 below. Also, the temperature-thermal shrinkage stress measurement curves are shown in Fig. 5 and Fig. 6.

[Table 1]

| | Spinning conditions | | | | | | |
| | PET material | Spinneret surface temperature | Spinneret surface temperature distribution | Hot zone[*1] length | Cooling conditions | | |
| | Reduced viscosity | | | | Cooling air temperature[*2] | Cooling air speed | Speed variation Z[*3] |
| | dl/g | °C | °C | mm | °C | m/s | |
| Reference Example 1 | 1.26 | 303 | 4 | 30 | 15 | 1.0 | 0.07 |
| Reference Example 2 | 1.16 | 296 | 3 | 25 | 15 | 1.0 | 0.07 |
| Reference Comp. Example 1 | 0.58 | 280 | 3 | Unmeasurable | 15 | 1.0 | 0.07 |
| Reference Comp. Example 2 | 0.60 | 275 | 3 | Unmeasurable | 15 | 1.0 | 0.07 |

[*1]Hot zone: Region with atmosphere temperature controlled to 150°C or higher (distance from spinneret surface center section in vertical direction)
[*2]Cooling air temperature: Temperature of cooling air blown from cooling air blower (temperature adjustment of cooling air using Thermo Heater)
[*3]Speed variation Z: Value of variation in cooling rate blown from the cooling air blowing side, expressed as standard deviation

EP 3 045 574 B1

[Table 2]

| | Fiber physical properties | | | | | |
|---|---|---|---|---|---|---|
| | Reduced viscosity | Total fineness | Single fiber fineness | Tensile strength | Tensile elongation | Thermal shrinkage stress maximum |
| | dl/g | dtex | dtex | cN/dtex | % | cN/dtex |
| Reference Example 1 | 0.982 | 20.0 | 0.13 | 5.3 | 27 | 0.31 |
| Reference Example 2 | 0.932 | 20.0 | 0.13 | 4.8 | 31 | 0.38 |
| Reference Comp. Example 1 | 0.580 | 20.0 | 0.13 | 3.0 | 13 | 0.18 |
| Reference Comp. Example 2 | 0.600 | 20.0 | 0.13 | 3.4 | 17 | 0.12 |

[Examples 1 and 2 and Comparative Examples 1 and 2]

The ultrafine polyester fibers of Reference Examples 1 and 2 and Reference Comparative Examples 1 and 2 were used as warp yarn and weft yarn to fabricate a plain weave tubular seamless woven fabric with an inner diameter of 50 mm (warp density: 72.8/cm (185/inch), weft density: 61.4/cm (156/inch)). The woven fabrics were subjected to scouring and heat setting under the following conditions, and a tubular seamless woven fabric with a 100 cm length, a Z-shaped stent of the same diameter (Nitinol, wire diameter: 0.33 mm) and a tapered 3/8 needle were used, with a stitch spacing of 5 mm and with the stent situated in the lengthwise direction of the fabric at a spacing of 10 mm, to fabricate a stent graft. In Examples 1 and 2 and Comparative Examples 1 and 2, looseness was seen between the stent and graft, several locations of gaps exceeding 2 mm being present between the stent and graft, with many being observed at both ends of the stent graft where it is particularly difficult to perform suturing. The stent grafts were subjected to sterilization treatment for finishing.

(Scouring conditions)

- 1 hour of rinsing in 98°C aqueous sodium carbonate (concentration: 1 g/l).
- 1 hour of rinsing in ultrapure water at 98°C.
- Drying for fixed period of time at room temperature, in both axial directions.

(Heat setting conditions)

- The scoured and dried fabric was set on a stainless steel core rod with $\phi$50 mm $\times$ 200 mm length, and set in a thermostatic bath at 180°C for 30 minutes.

(Sterilization conditions)

- Heat treatment for 30 minutes in a thermostatic bath at 185°C.

In Examples 1 and 2, integration between the stent and graft increased, and gaps between the stent and graft had disappeared. In Comparative Examples 1 and 2, however, no improvement in integration was seen from before sterilization treatment, and gaps between the stent and graft exceeding 2 mm still remained at numerous locations.

The weft yarn was pulled out from the fabrics and the physical properties such as thermal shrinkage stress were evaluated. The results are shown in Table 3 below. The temperature-thermal shrinkage stress curves are shown in Figs. 3 to 6.

The thermal shrinkage stress of the weft yarns of Examples 1 and 2 which had improved integration between stent and graft exceeded 0.05 cN/dtex, whereas the thermal shrinkage stress of the weft yarns of Comparative Examples 1 and 2 was lower than 0.05 cN/dtex.

[Table 3]

| | Integration of stent and graft[*1] | | Physical properties of graft weft yarn after sterilization | | | | | | | | | | | | |
|---|---|---|---|---|---|---|---|---|---|---|---|---|---|---|---|
| | Before sterilization | After sterilization | Total fineness | Single fiber fineness | Tensile strength | Tensile elongation | Thermal shrinkage stress maximum | Alkali etching treatment time | Alkali etching weight retention $Y_1/Y_0$ | Degree of crystallization before alkali etching $X_1$ | Degree of crystallization after alkali etching $X_2$ | Surface layer section degree of crystallization $X_3$ | Birefringence before alkali etching $\Delta n_t$ | Birefringence after alkali etching $\Delta n_c$ | Surface layer section birefringence $\Delta n_s$ |
| | Number | Number | dtex | dtex | cN/dtex | % | cN/dtex | hr | | % | % | % | % | % | % |
| Example 1 | 11 | 2 | 20.3 | 0.14 | 4.3 | 18 | 0.13 | 2.25 | 0.89 | 43.1 | 43.5 | 43.9 | 0.202 | 0.198 | 0.260 |
| Example 2 | 13 | 1 | 20.4 | 0.19 | 4.1 | 20 | 0.22 | 2.25 | 0.89 | 42.0 | 92.1 | 45.3 | 0.201 | 0.182 | 0.390 |
| Comp. Example 1 | 12 | 11 | 20.0 | 0.13 | 3.9 | 8 | 0.04 | 0.83 | 0.89 | 49.8 | 47.1 | 28.8 | 0.204 | 0.213 | 0.149 |
| Comp. Example 2 | 14 | 11 | 20.0 | 0.13 | 3.6 | 10 | 0.03 | 0.92 | 0.89 | 99.7 | 46.5 | 33.2 | 0.210 | 0.216 | 0.178 |

[*1] Integration of stent and graft: Evaluated by the counted number of loose sections (gaps) of 2 mm or greater at both ends or the stent graft.

Industrial Applicability

Ultrafine polyester fiber essentially composed entirely of a PET component according to the invention does not invite concerns regarding residue from polymers other than the PET component or from solvents, as is the case with composite spun ultrafine fibers, and allows narrow wall thicknesses to be obtained for stent graft fabrics and artificial blood vessels, while also solving the problem of integration between the stent and graft that can lead to blood leakage. In addition, because it has excellent long-term durability in the body, it can be suitably utilized as a material for implantation into the human body, such as a stent graft fabric or artificial blood vessel.

**Claims**

1.   An ultrafine polyester fiber having a polyethylene terephthalate component content of 98 wt% or greater, and satisfying the following features:

(1) a reduced viscosity ($\eta$sp/c) of 0.80 dl/g or greater,
(2) a total fineness of 7 dtex or greater and 120 dtex or less, and a single fiber fineness of 0.5 dtex or less,
(3) a maximum thermal shrinkage stress of 0.05 cN/dtex or greater in a temperature range of between 80°C and 200°C, and
(5) a birefringence of 0.20 or greater in the region spreading from the surface of the fiber to a depth of 0.1 $\mu$m;

wherein (1), (2), (3) and (5) are measured as indicated in the description.

2.   The ultrafine polyester fiber according to claim 1, further satisfying the following condition:

(4) a degree of crystallinity of 35% or greater in the region spreading from the surface of the fiber to a depth of 0.1 $\mu$m;

wherein (4) is measured as indicated in the description.

3.   A fabric comprising at least 20 wt% of the ultrafine polyester fiber according to claim 1 or 2.

4.   A stent graft fabric comprising at least 20 wt% of the ultrafine polyester fiber according to claim 1 or 2.

5.   A stent graft comprising the stent graft fabric according to claim 4.

6.   An artificial fiber fabric comprising at least 20 wt% of the ultrafine polyester fiber according to claim 1 or 2.

**Patentansprüche**

1.   Ultrafeine Polyesterfaser, die einen Gehalt an Polyethylenterephthalatkomponente von 98 Gew.-% oder mehr aufweist und den folgenden Merkmalen genügt:

(1) einer reduzierten Viskosität ($\eta$sp/c) von 0,80 dl/g oder mehr;
(2) einer Gesamtfeinheit von 7 dtex oder mehr und 120 dtex oder weniger und einer Einzelfaserfeinheit von 0,5 dtex oder weniger;
(3) einer maximalen thermischen Schrumpfungsspannung von 0,05 cN/dtex oder mehr in einem Temperaturbereich zwischen 80 °C und 200 °C; und
(5) einer Doppelbrechung von 0,20 oder mehr im Bereich, der sich von der Oberfläche der Faser bis zu einer Tiefe von 0,1 $\mu$m erstreckt;

wobei (1), (2), (3) und (5) so gemessen werden, wie es in der Beschreibung angegeben ist.

2.   Ultrafeine Polyesterfaser gemäß Anspruch 1, die weiterhin der folgenden Bedingung genügt:

(4) einem Grad der Kristallinität von 35% oder mehr im Bereich, der sich von der Oberfläche der Faser bis zu einer Tiefe von 0,1 $\mu$m erstreckt;

wobei (4) so gemessen wird, wie es in der Beschreibung angegeben ist.

**3.** Textilstoff, der wenigstens 20 Gew.-% der ultrafeinen Polyesterfaser gemäß Anspruch 1 oder 2 umfasst.

**4.** Stentimplantatstextilstoff, der wenigstens 20 Gew.-% der ultrafeinen Polyesterfaser gemäß Anspruch 1 oder 2 umfasst.

**5.** Stentimplantat, das den Stentimplantatstextilstoff gemäß Anspruch 4 umfasst.

**6.** Künstlicher Fasertextilstoff, der wenigstens 20 Gew.-% der ultrafeinen Polyesterfaser gemäß Anspruch 1 oder 2 umfasst.

**Revendications**

**1.** Fibre de polyester ultrafine ayant une teneur en constituant polyéthylène téréphtalate de 98 % en masse ou supérieure, et satisfaisant les aspects suivants :

(1) une viscosité réduite ($\eta$sp/c) de 0,80 dl/g ou supérieure,
(2) une finesse totale de 7 dtex ou supérieure et de 120 dtex ou inférieure, et une finesse de fibre unique de 0,5 dtex ou inférieure,
(3) une contrainte de retrait thermique maximale de 0,05 cN/dtex ou supérieure dans un intervalle de température de 80°C à 200°C, et
(5) une biréfringence de 0,20 ou supérieure dans la région s'étalant de la surface de la fibre jusqu'à une profondeur de 0,1 $\mu$m ;

où (1), (2), (3) et (5) sont mesurées comme indiqué dans la description.

**2.** Fibre de polyester ultrafine selon la revendication 1, satisfaisant de plus la condition suivante :

(4) un degré de cristallinité de 35 % ou supérieur dans la région s'étalant de la surface de la fibre jusqu'à une profondeur de 0,1 $\mu$m ;

où (4) est mesuré comme indiqué dans la description.

**3.** Textile comprenant au moins 20 % en masse de la fibre de polyester ultrafine selon la revendication 1 ou 2.

**4.** Textile de greffe d'endoprothèse comprenant au moins 20 % en masse de la fibre de polyester ultrafine selon la revendication 1 ou 2.

**5.** Greffe d'endoprothèse comprenant le textile de greffe d'endoprothèse selon la revendication 4.

**6.** Textile de fibre artificielle comprenant au moins 20 % en masse de la fibre de polyester ultrafine selon la revendication 1 ou 2.

FIG. 1

Intravascular wall
(circular solid line)

Stent
(circular dotted line)

Graft

Gap

Graft lengthwise
direction

# FIG. 2

Intravascular wall
(circular solid line)

Stent
(circular dotted line)

Graft

Graft lengthwise
direction

EP 3 045 574 B1

# FIG. 3

Reference Example 1, Example 1

# FIG. 4

Reference Example 2, Example 2

# FIG. 5

Reference Comparative Example 1, Comparative Example 1

# FIG. 6

Reference Comparative Example 2, Comparative Example 2

**EP 3 045 574 B1**

**REFERENCES CITED IN THE DESCRIPTION**

**Patent documents cited in the description**

- JP 55001338 A
- JP 55132708 A
- JP 2006132027 A

**Non-patent literature cited in the description**

- Fiber Handbook - Starting materials. Maruzen Publishing, 1978, 969